(19) Europäisches Patentamt
European Patent Office
Office européen des brevets

(11) Veröffentlichungsnummer: **0 036 841**
**A1**

# (12) EUROPÄISCHE PATENTANMELDUNG

(21) Anmeldenummer: **81810109.9**

(22) Anmeldetag: **20.03.81**

(51) Int. Cl.³: **C 07 D 339/06**, A 01 N 43/28
// C07C155/08

(30) Priorität: **26.03.80 CH 2373/80**

(43) Veröffentlichungstag der Anmeldung: **30.09.81**
**Patentblatt 81/39**

(84) Benannte Vertragsstaaten: **CH DE FR GB IT LI**

(71) Anmelder: **CIBA-GEIGY AG, Patentabteilung Postfach, CH-4002 Basel (CH)**

(72) Erfinder: **Fäh, Hansjakob, Dr., Schlossweg, CH-4466 Ormalingen (CH)**
Erfinder: **Grieder, Alfred, Dr., Ob den Reben 15, CH-4461 Böckten (CH)**
Erfinder: **Scheuzger, Karl, Markgräflerstrasse 71, CH-4057 Basel (CH)**

## (54) Verfahren zur Herstellung von 1,3-Benzodithiolderivaten.

(57) Es wird ein Verfahren zur Herstellung von 1,3-Benzodithiolderivaten der Formel

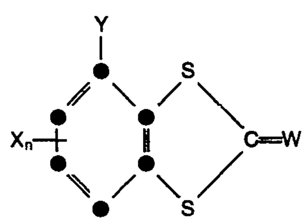

in welcher Y Nitro, Trifluormethyl, Cyano oder Alkylsulfonyl, X Alkyl, Alkenyl, Nitro, Trichlormethyl, Trifluormethyl, Trifluormethoxy, Trifluormethylthio, Trifluormethylsulfinyl, Trifluormethylsulfonyl, Methoxymethyl, Cyano, Carboxy, Carbamoyl, Halogen, Amino, Acylamino, N-Alkylamino, N,N-Dialkylamino, N-Phenylamino, N-Alkenylamino, N,N-Dialkenylamino, n 0, 1, 2 oder 3 und W Sauerstoff oder Schwefel bedeuten, beschrieben. Nach diesem Verfahren wird ein 2-Nitrophenyl-N,N-dialkyldithiocarbamat der Formel

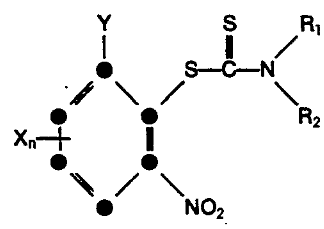

in welcher X, Y und n die unter Formel I angegebene Bedeutung haben und $R_1$ und $R_2$ je eine Alkylgruppe bedeuten, in Gegenwart einer starken Säure und eines inerten Lösungsmittels zunächst in ein Iminiumsalz der Formel

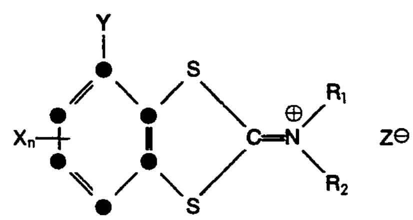

in welcher $R_1$, $R_2$, X, Y und n die oben angegebene Bedeutung haben und Z für das Anion einer starken Säure

EP 0 036 841 A1

steht, überführt und dieses anschliessend einer Verbindung der Formel

H—W—H

in welcher W Sauerstoff oder Schwefel bedeutet, zu einem 1,3-Benzodithiolderivat der obigen Formel umsetzt.

Ferner werden neue 1,3-Benzodithiol-2-N,N-dialkyliminiumsalze und ihre Herstellung beschrieben.

Die nach dem neuen Verfahren herstellbaren 1,3-Benzodithiolderivate können als Pestizide verwendet werden.

0036841

CIBA-GEIGY AG
Basel (Schweiz)

5-12777/=

Verfahren zur Herstellung von 1,3-Benzodithiolderivaten.

Die vorliegende Erfindung betrifft ein Verfahren zur Herstellung von 1,3-Benzodithiolderivaten der Formel I

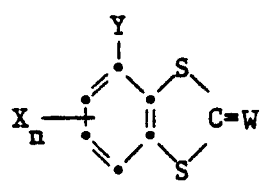

(I) ,

in welcher Y Nitro, Trifluormethyl, Cyano oder Alkylsulfonyl mit 1 bis 6 Kohlenstoffatomen, X Alkyl mit 1 bis 6 Kohlenstoffatomen oder Alkenyl mit 2 bis 6 Kohlenstoffatomen, Nitro, Trichlormethyl, Trifluormethyl, Trifluormethoxy, Trifluormethylthio, Trifluormethylsulfinyl, Trifluormethylsulfonyl, Methoxymethyl, Cyano, Carboxy, Carbamoyl, Halogen, Amino, Acylamino, N-Alkylamino mit 1 bis 6 Kohlenstoffatomen in der Alkylgruppe, N,N-Dialkylamino mit 1 bis 6 Kohlenstoffatomen in den Alkylgruppen, N-Phenylamino, N-Alkenylamino mit 3 bis 6 Kohlenstoffatomen in der Alkenylgruppe, N,N-Dialkenylamino mit 3 bis 6 Kohlenstoffatomen in den Alkenylgruppen, n 0, 1, 2 oder 3 und W Sauerstoff oder Schwefel bedeuten.

Die als bzw. in den Substituenten X und Y vorkommenden Alkyl- bzw. Alkenylgruppen können geradkettig oder verzweigt sein. Unter diesen Alkylgruppen sind solche mit 1 bis 4 Kohlenstoffatomen bzw. solche Alkenylgruppen mit 3 bis 4 Kohlenstoffatomen bevorzugt. Halogen bedeutet Fluor, Chlor, Brom oder Jod, vorzugsweise Chlor oder Brom.

Die 1,3-Benzodithiolderivate der Formel I sind biozid wirksame Verbindungen, deren Herstellung und Verwendung beispielsweise in den

deutschen Offenlegungsschriften 26 44 036 und 28 34 061 beschrieben ist.

Nach dem in der deutschen Offenlegungsschrift 26 44 036 beschriebenen Verfahren werden 1,3-Benzodithiol-2-one durch Umsetzung von 2-Halogennitrobenzolen mit Natrium-N,N-dialkyldithiocarbamat-dihydrat und unmittelbar anschliessende Zersetzung des in situ gebildeten 2-Nitrophenyl-N,N-dialkyldithiocarbamats hergestellt. Dabei werden die 1,3-Benzodithiol-2-one in einer Ausbeute von weniger als 50% d.Th. erhalten. Ausserdem wird ein nicht näher identifiziertes unlösliches Nebenprodukt erhalten, das unter alkalischen Bedingungen in das gewünschte 1,3-Benzodithiol-2-on überführt werden kann.

Nach einem weiteren, in J. Org. Chem. 44 (2), 267-274, (1979) beschriebenen Verfahren werden 1,3-Benzodithiol-2-one durch Zersetzung von 2-Nitrophenyl-N,N-dialkyldithiocarbamaten in Aceton, Dimethylsulfoxid und Dimethylformamid als Lösungsmittel hergestellt. Dabei betragen die Ausbeuten bestenfalls 75-80% d.Th. bezogen auf eingesetztes 2-Nitrophenyl-N,N-dialkyldithiocarbamat.

1,3-Benzodithiol-2-thione werden nach einem in der deutschen Offenlegungsschrift 26 44 036 beschriebenen Verfahren durch Umsetzung der entsprechenden 1,3-Benzodithiol-2-one mit einem Sulfurierungsmittel, wie Dinatriumtrithiocarbonat, Natriumhydrosulfid oder Kaliumthiocyanat erhalten. Ferner können 1,3-Benzodithiol-2-thione nach einem in der deutschen Offenlegungsschrift 28 34 061 beschriebenen Verfahren durch Umsetzung von entsprechenden 2-Halogennitrobenzolen mit Natrium-S-tert.butyl-trithiocarbonat zu 2-Nitrophenyl-S-tert.butyl-trithiocarbonaten und deren anschliessende Zersetzung hergestellt werden. Hierbei werden die 1,3-Benzodithiol-2-thione in einer Ausbeute von etwa 22% bezogen auf 2-Halogennitrobenzol und in einer Ausbeute von 55% bezogen auf 2-Nitrophenyl-S-tert.butyl-trithiocarbonat erhalten.

Die mit den bisher bekannten Verfahren erreichbaren Ausbeuten sind für eine technische Herstellung von 1,3-Benzodithiolderivaten

der Formel I unbefriedigend. Ausserdem haben die bekannten Verfahren den Nachteil, dass das Endprodukt stets nachweisbare Mengen an Nitrosamin enthält, das bei der Zersetzung des 2-Nitrophenyl-N,N-dialkyldithiocarbamats als Nebenprodukt gebildet wird. Dies macht einen weiteren Reinigungsschritt erforderlich, da das Nitrosamin wegen seiner cancerogenen Wirkung unbedingt entfernt werden muss.

Der vorliegenden Erfindung liegt daher die Aufgabe zugrunde, die Herstellung von nitrosaminfreien 1,3-Benzodithiolderivaten der Formel I auf einfache Weise und in guten Ausbeuten zu ermöglichen.

Gemäss vorliegender Erfindung wird vorgeschlagen, die 1,3-Benzodithiolderivate der Formel I in der Weise herzustellen, dass man ein 2-Nitrophenyl-N,N-dialkyldithiocarbamat der Formel II

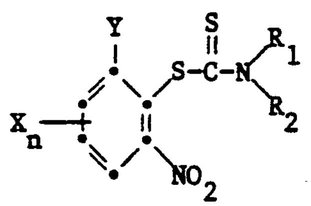

(II) ,

in welcher X, Y und n die unter Formel I angegebene Bedeutung haben, und $R_1$ und $R_2$ je eine Alkylgruppe mit 1 bis 6 Kohlenstoffatomen bedeuten, in Gegenwart einer starken Säure und eines inerten Lösungsmittels in ein Iminiumsalz der Formel III

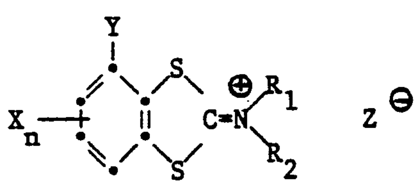

(III) ,

in welcher $R_1$, $R_2$, X, Y und n die oben angegebene Bedeutung haben, und Z für das Anion einer starken Säure steht, überführt und dieses anschliessend mit einer Verbindung der Formel IV

H - W - H (IV)

in welcher W Sauerstoff oder Schwefel bedeutet, zu einem 1,3-Benzodithiolderivat der Formel I umsetzt.

Als Verbindungen der Formel IV kommen Wasser und Schwefelwasserstoff in Betracht. Schwefelwasserstoff kann entweder direkt in das Reaktionsgemisch eingeleitet oder in Form von Verbindungen zugesetzt werden, aus denen leicht Schwefelwasserstoff freigesetzt werden kann. Als Verbindungen, aus denen leicht Schwefelwasserstoff freigesetzt werden kann, kommen in erster Linie Salze des Schwefelwasserstoffs, wie Alkali- und Erdalkalisulfide, z.B. Natriumsulfid, Kaliumsulfid und Calciumsulfid und insbesondere Alkali- und Erdalkalihydrosulfide, wie Natriumhydrosulfid, Kaliumhydrosulfid und Calciumhydrosulfid in Frage. Die Verbindungen der Formel IV werden in der Regel in wenigstens stöchiometrischer Menge eingesetzt, wobei Wasser in grösserem Ueberschuss vorhanden sein und als Lösungsmittel dienen kann.

Das erfindungsgemässe Verfahren kann mit oder ohne Isolierung der intermediär gebildeten Iminiumsalze der Formel III als Zweistufenverfahren oder als Einstufenverfahren durchgeführt werden.

Die Ueberführung der 2-Nitrophenyl-N,N-dialkyldithiocarbamate der Formel II in Iminiumsalze der Formel III erfolgt in der Regel bei Temperaturen von 0-200°C, vorzugsweise bei 20-100°C. Geeignete Lösungsmittel sind vor allem Dialkylketone mit je 1 bis 4 Kohlenstoffatomen in den Alkylgruppen und Alkylester niederaliphatischer Carbonsäuren mit 2 bis 4 Kohlenstoffatomen im Säureteil und 1 bis 4 Kohlenstoffatomen im Alkoholteil. Geeignete Dialkylketone sind insbesondere Aceton, Methyläthylketon, Diäthylketon und Methylisobutylketon. Als geeignete Carbonsäureester sind insbesondere Aethylacetat, Butylacetat, Aethylpropionat und Methylbutyrat zu erwähnen.

Als starke Säuren, deren Anion in Formel III durch Z symbolisiert wird, kommen allgemein starke Mineralsäuren und starke organische Säuren in Betracht. Geeignete Säuren sind vor allem Chlorwasserstoff, Bromwasserstoff, Jodwasserstoff, Schwefelsäure, Salpetersäure, Perchlorsäure, Phosphorsäure, Tetrafluorborsäure, Trifluoressigsäure und Trichloressigsäure. Bevorzugte Säuren sind Chlorwasserstoff, Schwefelsäure und Perchlorsäure. Die Säuren können als solche oder in Form ihrer wässrigen Lösungen im Konzentrationsbereich von 30-100 % eingesetzt werden. Die Verwendung von wässrigen Lösungen der Säuren bietet insbesondere im Zusammenhang mit der Abtrennung der Iminiumsalze der Formel III von Nebenprodukten und Verunreinigungen Vorteile und ist daher bevorzugt. Die Säuren werden in mindestens stöchiometrischer Menge, vorzugsweise jedoch in einem Ueberschuss von bis zu 10 Mol pro Mol 2-Nitrophenyl-N,N-dithiocarbamat der Formel II eingesetzt.

Zur Isolierung der Iminiumsalze der Formel III ist es vorteilhaft, in einem zweiphasigen, aus organischem Lösungsmittel und Wasser bestehenden Reaktionsmedium zu arbeiten. In diesem Fall wird nach Abtrennung des organischen Lösungsmittels eine wässrige Lösung des Iminiumsalzes der Formel III erhalten, aus der das Iminiumsalz, gegebenenfalls nach Neutralisation der überschüssigen Säure, in kristalliner Form isoliert werden kann.

Die Iminiumsalze der Formel III sind neue Verbindungen und ebenfalls Gegenstand der Erfindung.

Die Umsetzung der Iminiumsalze der Formel III mit einer Verbindung der Formel IV wird in der Regel bei Temperaturen von 0-150°C, vorzugsweise bei 10-100°C, in einem wässrigen Reaktionsmedium bei einem pH-Wert von in der Regel 0-10, vorzugsweise bei einem pH-Wert von 1-8 durchgeführt. Das Reaktionsmedium besteht entweder aus Wasser oder einem Gemisch aus Wasser und einem organischen Lösungsmittel. Dabei können sowohl organische Lösungsmittel, die mit Wasser nicht mischbar sind, als auch solche, die mit Wasser mischbar sind, verwendet werden. Geeignete organische Lösungsmittel sind beispielsweise Ketone, wie Aceton, Methyläthylketon und Methylisopropylketon, aromatische Kohlenwasserstoffe, wie Benzol, Toluol oder Chlorbenzol, sowie Dimethylsulfoxid und Dimethylformamid. Die Umsetzung nimmt je nach Reaktionstemperatur eine halbe bis 24 Stunden in Anspruch. In den meisten Fällen genügt eine Reaktionszeit von 1 bis 10 Stunden.

Bei Verwendung von Wasser als Reaktionsmedium wird das 1,3-Benzodithiolderivat der Formel I unmittelbar als kristallines Produkt erhalten, das leicht durch Filtration abgetrennt werden kann. Bei Verwendung eines aus Wasser und einem mit Wasser nicht mischbaren Lösungsmittel bestehenden Reaktionsmediums kann das 1,3-Benzodithiolderivat der Formel I als Lösung in dem verwendeten Lösungsmittel abgetrennt und durch Abdampfen des Lösungsmittels gewonnen werden. Bei Verwendung eines aus Wasser und einem mit Wasser mischbaren Lösungsmittel bestehenden Reaktionsmediums, aus dem sich das 1,3-Benzodithiolderivat der Formel I nicht oder nur teilweise abscheidet, kann die Isolierung in einfacher Weise durch Extraktion mit einem geeigneten, mit Wasser nicht mischbaren Lösungsmittel, wie Methylisobutylketon oder Toluol, erfolgen.

Gemäss einer vorteilhaften Ausführungsform des erfindungsgemässen Verfahrens wird ein 2-Nitrophenyl-N,N-dialkyldithiocarbamat der Formel II in einem aus Wasser und einem mit Wasser nicht mischbaren Lösungsmittel bestehenden Reaktionsmedium in Gegenwart von 1-10 Mol pro Mol 2-Nitrophenyl-N,N-dialkyldithiocarbamat der Formel II Chlorwasserstoff, Schwefelsäure oder Perchlorsäure bei einer Temperatur von 10-100° C in das Iminiumsalz der Formel III überführt, die organische Phase abgetrennt und das Iminiumsalz der Formel III aus der wässrigen Phase nach Neutralisation der überschüssigen Säure in kristalliner Form isoliert und anschliessend in einem wässrigen Reaktionsmedium bei pH 1-8 und einer Temperatur von 10-100°C mit Wasser oder Schwefelwasserstoff zu einem 1,3-Benzodithiolderivat der Formel I umgesetzt. Bei dieser Ausführungsform des erfindungsgemässen Verfahrens wird mit der Abtrennung des Iminiumsalzes der Formel III gleichzeitig eine

vollständige Abtrennung der bei der Bildung des Iminiumsalzes entstandenen salpetrigen Säure erreicht. Dadurch kann die anschliessende Umsetzung des Iminiumsalzes mit Wasser oder Schwefelwasserstoff in Abwesenheit von salpetriger Säure durchgeführt werden und das bei der Umsetzung gebildete Dialkylamin kann nicht zu Nitrosamin reagieren. Auf diese Weise wird ein nitrosaminfreies 1,3-Benzodithiolderivat der Formel I erhalten.

Die nach Abtrennung der Lösung des Iminiumsalzes der Formel III im Filtrat gelöste salpetrige Säure kann auf übliche Weise durch bekannte Reaktionen, wie Umsetzung mit Amidosulfonsäure, Harnstoff oder Hydroxylamin, durch Reaktion mit dem Lösungsmittel (Bildung von Isonitrosoverbindungen) oder durch Reduktion, beispielsweise mit schwefeliger Säure, vernichtet werden. Die Vernichtung der bei der Bildung der Iminiumsalze der Formel III entstehenden salpetrigen Säure kann auch in vorteilhafter Weise in situ erfolgen, indem man die Bildung der Iminiumsalze der Formel III in Gegenwart einer Substanz, die mit salpetriger Säure reagieren kann, wie Amidosulfonsäure, Harnstoff oder Hydroxylamin, oder in Gegenwart eines Reduktionsmittels, wie schwefelige Säure, durchführt und so die salpetrige Säure unmittelbar bei ihrer Entstehung abfängt.

Die vorgenannten Substanzen, die mit salpetriger Säure reagieren, werden in der Regel in wenigstens stöchiometrischer Menge, vorteilhaft in stöchiometrischer Menge bis zu einem Ueberschuss von 20% bezogen auf die stöchiometrische Menge, verwendet.

Gemäss einer weiteren vorteilhaften Ausführungsform des erfindungsgemässen Verfahrens wird ein 2-Nitrophenyl-N,N-dialkyldithiocarbamat der Formel II in einem aus Wasser und einem mit Wasser nicht mischbaren Lösungsmittel bestehenden Reaktionsmedium in Gegenwart von 1-10 Mol pro Mol 2-Nitrophenyl-N,N-dialkyldithiocarbamat der Formel II Chlorwasserstoff, Schwefelsäure oder Perchlorsäure bei einer Temperatur von 10-100° C in das entsprechende Iminiumsalz der Formel III überführt, die organische Phase abgetrennt und das in wässriger Lösung

vorliegende Iminiumsalze nach Zugabe von frischem organischem Lösungsmittel und Neutralisation der überschüssigen Säure in Gegenwart von Amidosulfonsäure, Harnstoff, Hydroxylamin oder schwefeliger Säure bei 10-100°C und einem pH von 1-8 mit einer Verbindung der Formel IV zum gewünschten 1,3-Benzodithiolderivat der Formel I umsetzt.

Bei dieser Ausführungsform des erfindungsgemässen Verfahrens wird vorteilhaft Methylisobutylketon als Lösungsmittel verwendet. Die mit salpetriger Säure reagierenden Substanzen Amidosulfonsäure, Harnstoff, Hydroxylamin und schwefelige Säure werden in wenigstens stöchiometrischer Menge eingesetzt, wobei schwefelige Säure vorteilhaft in Form von Natriumhydrogensulfit zur Anwendung kommt. Vorzugsweise verwendet man diese Substanzen in einem Ueberschuss von 5 - 200 % bezogen auf die stöchiometrische Menge. Unter den Substanzen, in deren Gegenwart die Umsetzung des Iminiumsalzes der Formel III mit Wasser oder Schwefelwasserstoff durchgeführt wird, ist Natriumhydrogensulfit bevorzugt.

Bei der letztgenannten Ausführungsform des erfindungsgemässen Verfahrens wird durch die Gegenwart von Substanzen, die mit salpetriger Säure reagieren können, die bei der Bildung des Iminiumsalzes der Formel III gebildete salpetrige Säure abgefangen, bzw. das aus salpetriger Säure und dem bei der Umsetzung mit Wasser oder Schwefelwasserstoff freigesetzten Dialkylamin entstandene Nitrosamin reduziert. Auf diese Weise wird ein nitrosaminfreies 1,3-Benzodithiolderivat der Formel I erhalten.

Die nach dem erfindungsgemässen Verfahren herstellbaren 1,3-Benzodithiolderivate der Formel I sind, wie eingangs bereits ausgeführt, biozide Substanzen. Sie können beispielsweise als Herbizide, Fungizide, Akarizide, Nematozide und Insektizide eingesetzt werden.

Die als Ausgangsmaterialien benötigten 2-Nitrophenyl-N,N-dialkyldithiocarbamate der Formel II können durch Umsetzung der entsprechenden 2-Nitrochlorphenole mit Natrium-N,N-dialkyldithiocarbamat hergestellt werden (vgl. J. Org. Chem. 44 (2), 272 (1979)). Durch das erfindungsgemässe Verfahren können nitrosaminfreie 1,3-Benzodithiol-2-one der Formel I durch Zersetzung von 2-Nitrophenyl-N,N-dialkyldithiocarbamaten auf einfache Weise und in guten Ausbeuten hergestellt werden.

Das erfindungsgemässe Verfahren wird durch die folgenden Beispiele näher erläutert.

Beispiel 1: Herstellung von 4-n-Propylamino-5-nitro-7-trifluormethyl-1,3-benzodithiol-2-N,N-dimethyliminiumhydrogensulfat.

a) In einem mit Rührer, Thermometer und Kühler versehenen Reaktionskolben wird zu einer Mischung von 206 g (0,5 Mol) 2,4-Dinitro-3-n-propylamino-6-trifluormethylphenyl-N,N-dimethyldithiocarbamat und 450 g Methylisobutylketon innerhalb von 30 Minuten bei 20-30° C 350 g 70%-ige Schwefelsäure (2,5 Mol) zudosiert. Anschliessend wird das Reaktionsgemisch innerhalb 1 Stunde auf 60-65° C aufgeheizt und dann 4 Stunden bei 60-65° C gerührt. Nach dem Abkühlen auf Raumtemperatur und Zugabe von 90 ml Wasser wird die obere organische Phase abgetrennt. Zur wässrigen Phase werden unter Rühren bei 0-5° C während 1 Stunde 160 g 50%-ige Natronlauge (2,0 Mol) zugefügt. Die abgeschiedenen Kristalle werden abfiltriert, mit wenig kaltem Wasser gewaschen und im Vakuumtrockenschrank bei 60° C getrocknet. Es werden 220 g (95% d.Th.) 4-n-Propylamino-5-nitro-7-trifluormethyl-1,3-benzodithiol-2-N,N-dimethyliminiumhydrogensulfat vom Schmelzpunkt 180-182° C erhalten.

b) Herstellung von 4-n-Propylamino-5-nitro-7-trifluormethyl-1,3-benzodithiol-2-on.

In einem mit Rührer, Thermometer und Kühler versehenen Reaktionskolben werden 185 g (0,4 Mol) 4-n-Propylamino-5-nitro-7-trifluormethyl-1,3-benzodithiol-2-N,N-dimethyliminiumhydrogensulfat und 600 ml Wasser innerhalb von 1 Stunde auf Rückflusstemperatur aufgeheizt und 5 Stunden bei dieser Temperatur gehalten. Nach dem Abkühlen des Reaktionsgemisches auf 20-30° C wird das auskristallisierte 4-n-Propylamino-5-nitro-7-trifluormethyl-1,3-benzodithiol-2-on abfiltriert, mit Wasser gewaschen und im Vakuumtrockenschrank bei 40° C getrocknet. Ausbeute: 132 g (98% d.Th.), Schmelzpunkt: 81-83° C.

c) Herstellung von 4-n-Propylamino-5-nitro-7-trifluormethyl-1,3-benzodithiol-2-on.

In einem mit Rührer, Thermometer und Kühler versehenen Reaktionskolben werden 23,0 g (0,05 Mol) 4-n-Propylamino-5-nitro-7-trifluormethyl-1,3-benzodithiol-2-N,N-dimethyliminiumhydrogensulfat, 100 ml Wasser und 100 g Methylisobutylketon innerhalb 1 Stunde auf 80° C aufgeheizt und 10 Stunden bei dieser Temperatur gehalten. Anschliessend wird das Reaktionsgemisch auf Raumtemperatur abgekühlt und die obere organische Phase abgetrennt. Nach Abtrennung des Lösungsmittels im Rotationsverdampfer und Trocknen des Destillationsrückstands im Vakuumtrockenschrank bei 40° C werden 16,0 g (95% d.Th.) 4-n-Propylamino-5-nitro-7-trifluormethyl-1,3-benzodithiol-2-on vom Schmelzpunkt 80-83° C erhalten.

Beispiel 2: Herstellung von 4-n-Propylamino-5-nitro-7-trifluormethyl-1,3-benzodithiol-2-N,N-dimethyliminiumperchlorat.

In einem mit Rührer, Thermometer und Rückflusskühler versehenen Reaktionskolben werden 20,6 g (0,05 Mol) 2,4-Dinitro-3-n-propylamino-6-trifluormethyl-N,N-dimethyldithiocarbamat und 2,0 g Methylisobutylketon vorgelegt und innerhalb von 30 Minuten 33 g (0,23 Mol) 70%-ige Perchlorsäure bei 60-65° C zudosiert. Dann wird das Reaktionsgemisch abgekühlt und bei 0-5° C zunächst mit 20 g Wasser und dann mit 15,1 g (0,18 Mol) 50%-iger Natronlauge versetzt. Die abgeschiedenen Kristalle werden abfiltriert, mit wenig kaltem Wasser gewaschen und im Vakuumtrockenschrank bei 40° C getrocknet. Es werden so 22,0 g (95% d. Th.) 4-n-Propylamino-5-nitro-7-trifluormethyl-1,3-benzodithiol-2-N,N-dimethyliminiumperchlorat vom Schmelzpunkt 153-156° C erhalten.

Bei der Hydrolyse des 4-n-Propylamino-5-nitro-7-trifluormethyl-1,3-benzodithiol-2-N,N-dimethyliminiumperchlorats nach den in Beispiel 1 beschriebenen Methoden wird 4-n-Propylamino-5-nitro-7-trifluormethyl-1,3-benzodithiol-2-on in gleich guter Ausbeute erhalten.

Beispiel 3: Herstellung von 4-n-Propylamino-5-nitro-7-trifluormethyl-1,3-benzodithiol-2-N,N-dimethyliminiumperchlorat.

In einem mit Rührer, Thermometer und Kühler versehenen Reaktionskolben werden bei 20-30° C zu einer Mischung von 20,6 g (0,05 Mol) 2,4-Dinitro-3-n-propylamino-6-trifluormethylphenyl-N,N-dimethyldithiocarbamat und 60 g Methylisobutylketon innerhalb von 30 Minuten 60 g (0,53 Mol) 32%-ige Salzsäure zudosiert. Nach beendigter Zugabe der Salzsäure wird das Reaktionsgemisch innerhalb von 1 Stunde auf 55-60° C aufgeheizt und anschliessend 3 Stunden bei dieser Temperatur gehalten. Nach dem Abkühlen auf 20-25° C und Zugabe von 60 ml Wasser wird die obere organische Phase abgetrennt. Die wässrige Phase wird innerhalb 1 Stunde bei 0-5° C mit 33,0 g (0,23 Mol) 70%-iger Perchlorsäure versetzt. Die abgeschiedenen Kristalle werden abfiltriert, mit wenig kaltem Wasser gewaschen und im Vakuumtrockenschrank bei 40° C getrocknet. Es werden 16,2 g (70% d.Th.) 4-n-Propylamino-5-nitro-7-trifluormethyl-1,3-benzodithiol-2-N,N-dimethyliminiumperchlorat vom Schmelzpunkt 153-156° C erhalten.

Beispiel 4: Herstellung von 4-n-Propylamino-5-nitro-7-trifluormethyl-1,3-benzodithiol-2-on.

In einem mit Rührer, Thermometer und Kühler versehenen Reaktionskolben werden zu einer Mischung von 206 g (0,5 Mol) 2,4-Dinitro-3-n-propylamino-6-trifluormethylphenyl-N,N-dimethyldithiocarbamat und 450 g Methylisobutylketon innerhalb von 30 Minuten 350 g (2,5 Mol) 70%-ige Schwefelsäure zudosiert. Anschliessend wird das Reaktionsgemisch innerhalb 1 Stunde auf 60-65° C aufgeheizt und 4 Stunden bei dieser Temperatur gehalten. Nach Abkühlung auf Raumtemperatur und Zugabe von 90 ml Wasser wird die obere organische Phase abgetrennt. Zur wässrigen Phase werden nach Zugabe von 450 g Methylisobutylketon bei 20-25° C unter Rühren innerhalb 1 Stunde 160 g (2,0 Mol) 50%-ige Natronlauge zudosiert. Die erhaltene Mischung wird mit 320 g einer

40%-igen Natriumhydrogensulfitlösung versetzt und anschliessend innerhalb 1 Stunde auf 55-60° C aufgeheizt und dann 4 Stunden bei dieser Temperatur gehalten. Danach wird das Reaktionsgemisch auf 20-30° C abgekühlt und die obere organische Phase abgetrennt. Nach Abdampfen des Lösungsmittels im Rotationsverdampfer und Umkristallisation des Destillationsrückstandes aus der 4-fachen Gewichtsmenge Aethanol werden 144 g (80% d.Th.) 4-n-Propylamino-5-nitro-7-trifluormethyl-1,3-benzodithiol-2-on vom Schmelzpunkt 80-83° C erhalten.

Beispiel 5: In einem mit Rührer, Thermometer und Kühler versehenen Reaktionskolben werden 206 g (0,5 Mol) 2,4-Dinitro-3-n-propylamino-6-trifluormethylphenyl-N,N-dimethyldithiocarbamat und 450 g Methylisobutylketon vorgelegt. Nach Zugabe von 250 ml Wasser werden innerhalb von 30 Minuten bei 20-30° C 600 g einer 40%-igen Natriumhydrogensulfitlösung und anschliessend ebenfalls innerhalb von 30 Minuten bei 20-30° C 170 g 32%-ige Salzsäure eingerührt, bis ein pH-Wert von 1 erreicht ist. Das Reaktionsgemisch wird dann innerhalb von 1 Stunde auf 55-60° C aufgeheizt, 2 Stunden bei dieser Temperatur gehalten und auf 20-30° C abgekühlt. Nach Abtrennung der wässrigen Phase wird die organische Phase am Rotationsverdampfer eingedampft. Der Destillationsrückstand wird aus der 4-fachen Gewichtsmenge Aethanol umkristallisiert. Es werden 101 g (60% d.Th.) 4-n-Propylamino-5-nitro-7-trifluormethyl-1,3-benzodithiol-2-on vom Schmelzpunkt 81-83° C erhalten.

Beispiel 6: Herstellung von 2,4-Dinitro-3-n-propylamino-6-trifluormethylphenyl-N,N-dimethyldithiocarbamat.

In einem mit Rührer, Thermometer und Kühler versehenen Reaktionskolben werden bei 0-5° C 225 g (1,25 Mol) Natrium-N,N-dimethyldithiocarbamatdihydrat, 375 ml Wasser, 500 g Methylisobutylketon und 2,0 g Tetrabutylammoniumbromid vorgelegt. In diese Mischung wird innerhalb von 2 Stunden unter Rühren bei 0-5° C eine Lösung von 409 g (1,25 Mol) 2-Chlor-3,5-dinitro-4-n-propylaminobenzotrifluorid in 1000 g

Methylisobutylketon eindosiert. Nach beendigter Zugabe wird das Reaktionsgemisch weitere 2 Stunden bei 0-5° C gerührt und anschliessend bei der gleichen Temperatur filtriert. Das erhaltene Produkt wird mit 300 ml kaltem Wasser gewaschen und im Vakuumtrockenschrank bei 30° C getrocknet. Man erhält 470 g (91% d.Th.) 2,4-Dinitro-3-n-propylamino-6-trifluormethylphenyl-N,N-dimethyldithiocarbamat vom Schmelzpunkt 110-112° C.

Beispiel 7: Herstellung von 4-n-Propylamino-5-nitro-7-trifluormethyl-1,3-benzodithiol-2-thion.

In einem mit Rührer, Thermometer und Kühler versehenen Reaktionskolben werden 11,6 g (0,025 Mol) 4-n-Propylamino-5-nitro-7-trifluormethyl-1,3-benzodithiol-2-N,N-dimethyliminiumhydrogensulfat, 80 g Methylisobutylketon, 50 ml Wasser und 1,3 g (0,013 Mol) 100%-ige Schwefelsäure vorgelegt. In diese Mischung wird innerhalb von 60 Minuten bei 20-30° C eine Lösung von 2,0 g (0,026 Mol) Natriumhydrogensulfid-Monohydrat in 50 ml Wasser eingetropft. Anschliessend wird die Reaktionsmischung weitere 2 Stunden bei 20-30° C gerührt. Dann wird die wässrige Phase abgetrennt und die organische Phase zweimal mit je 30 ml Wasser extrahiert. Nach dem Abdestillieren des Lösungsmittels im Vakuum und Trocknen des Destillationsrückstandes im Vakuumtrockenschrank bei 50° C werden 6,3 g (71% d.Th.) 4-n-Propylamino-5-nitro-7-trifluormethyl-1,3-benzodithiol-2-thion vom Schmelzpunkt 96-99° C erhalten.

Patentansprüche

1. Verfahren zur Herstellung von 1,3-Benzodithiolderivaten der Formel I

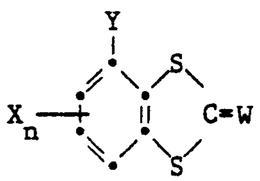

(I) ,

in welcher Y Nitro, Trifluormethyl, Cyano oder Alkylsulfonyl mit 1 bis 6 Kohlenstoffatomen, X Alkyl mit 1 bis 6 Kohlenstoffatomen oder Alkenyl mit 2 bis 6 Kohlenstoffatomen, Nitro, Trichlormethyl, Trifluormethyl, Trifluormethoxy, Trifluormethylthio, Trifluormethylsulfinyl, Trifluormethylsulfonyl, Methoxymethyl, Cyano, Carboxy, Carbamoyl, Halogen, Amino, Acylamino, N-Alkylamino mit 1 bis 6 Kohlenstoffatomen in der Alkylgruppe, N,N-Dialkylamino mit 1 bis 6 Kohlenstoffatomen in den Alkylgruppen, N-Phenylamino, N-Alkenylamino mit 3 bis 6 Kohlenstoffatomen in der Alkenylgruppe, N,N-Dialkenylamino mit 3 bis 6 Kohlenstoffatomen in den Alkenylgruppen, n 0, 1, 2 oder 3 und W Sauerstoff oder Schwefel bedeuten, dadurch gekennzeichnet, dass man ein 2-Nitrophenyl-N,N-dialkyldithiocarbamat der Formel II

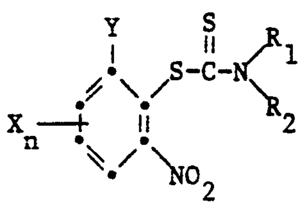

(II) ,

in welcher X, Y und n die unter Formel I angegebene Bedeutung haben, und $R_1$ und $R_2$ je eine Alkylgruppe mit 1 bis 6 Kohlenstoffatomen bedeuten, in Gegenwart einer starken Säure und eines inerten Lösungsmittels in ein Iminiumsalz der Formel III

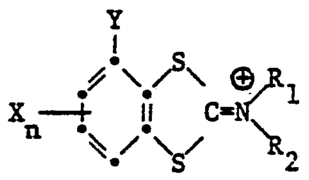

$Z^{\ominus}$ (III) ,

in welcher $R_1$, $R_2$, X, Y und n die oben angegebene Bedeutung haben, und Z für das Anion einer starken Säure steht, überführt und dieses anschliessend mit einer Verbindung der Formel IV

$$H - W - H \qquad (IV)$$

in welcher W Sauerstoff oder Schwefel bedeutet, zu einem 1,3-Benzodithiolderivat der Formel I umsetzt.

2. Verfahren nach Anspruch 1, dadurch gekennzeichnet, dass man die Ueberführung des 2-Nitrophenyl-N,N-dialkyldithiocarbamats der Formel II in das Iminiumsalz der Formel III bei einer Temperatur von 0-200° C vornimmt.

3. Verfahren nach Anspruch 1, dadurch gekennzeichnet, dass man die Ueberführung des 2-Nitrophenyl-N,N-dialkyldithiocarbamats der Formel II in das Iminiumsalz der Formel III bei einer Temperatur von 20-100° C vornimmt.

4. Verfahren nach Anspruch 1, dadurch gekennzeichnet, dass man als starke Säure eine starke Mineralsäure oder eine starke organische Säure verwendet.

5. Verfahren nach Ansprüchen 1 und 4, dadurch gekennzeichnet, dass man als starke Säure Chlorwasserstoff, Bromwasserstoff, Jodwasserstoff, Schwefelsäure, Salpetersäure, Perchlorsäure, Phosphorsäure, Tetrafluorborsäure, Trifluoressigsäure oder Trichloressigsäure verwendet.

6. Verfahren nach Ansprüchen 1, 4 und 5, dadurch gekennzeichnet, dass man als starke Säure Chlorwasserstoff, Schwefelsäure oder Perchlorsäure verwendet.

7. Verfahren nach Ansprüchen 1 und 4, dadurch gekennzeichnet, dass man die starke Säure in Form ihrer wässrigen Lösung im Konzentrationsbereich von 30-100% verwendet.

8. Verfahren nach Ansprücheu 1 und 4, dadurch gekennzeichnet, dass man die starke Säure in einem Ueberschuss von bis zu 10 Mol pro Mol 2-Nitrophenyl-N,N-dialkyldithiocarbamats der Formel II einsetzt.

9. Verfahren nach Anspruch 1, dadurch gekennzeichnet, dass man die Ueberführung des 2-Nitrophenyl-N,N-dialkyldithiocarbamats der Formel II in ein Iminiumsalz der Formel III in Gegenwart eines Dialkylketons mit je 1 bis 4 Kohlenstoffatomen in den Alkylgruppen oder in Gegenwart eines Alkylesters einer niederaliphatischen Carbonsäure mit 2 bis 4 Kohlenstoffatomen im Säureteil und 1 bis 4 Kohlenstoffatomen im Alkoholteil vornimmt.

10. Verfahren nach Ansprüchen 1 und 9, dadurch gekennzeichnet, dass man die Ueberführung des 2-Nitrophenyl-N,N-dialkyldithiocarbamats der Formel II in das Iminiumsalz der Formel III in Gegenwart von Aceton, Methyläthylketon, Diäthylketon, Methylisobutylketon, Aethylacetat, Butylacetat, Aethylpropionat oder Methylbutyrat vornimmt.

11. Verfahren nach Anspruch 1, dadurch gekennzeichnet, dass man die Ueberführung eines 2-Nitrophenyl-N,N-dialkyldithiocarbamats der Formel II in ein Iminiumsalz der Formel III in einem zweiphasigen, aus organischem Lösungsmittel und Wasser bestehenden Reaktionsmedium durchführt.

12. Verfahren nach Anspruch 1, dadurch gekennzeichnet, dass man die Ueberführung eines 2-Nitrophenyl-N,N-dialkyldithiocarbamats der Formel II in ein Iminiumsalz der Formel III in Gegenwart einer Substanz vornimmt, die mit der bei dieser Reaktion gebildeten salpetrigen Säure reagiert.

13. Verfahren nach Ansprüchen 1 und 12, dadurch gekennzeichnet, dass man die Ueberführung eines 2-Nitrophenyl-N,N-dialkyldithiocarbamats der Formel II in ein Iminiumsalz der Formel III in Gegenwart von Amidosulfonsäure, Harnstoff, Hydroxylamin oder schwefeliger Säure durchführt.

14. Verfahren nach Anspruch 1, dadurch gekennzeichnet, dass man die Umsetzung eines Iminiumsalzes der Formel III mit einer Verbindung der Formel IV bei Temperaturen von 0-150° C in einem wässrigen Reaktionsmedium bei einem pH-Wert von 0-10 durchführt.

15. Verfahren nach Ansprüchen 1 und 14, dadurch gekennzeichnet, dass man die Umsetzung eines Iminiumsalzes der Formel III mit einer Verbindung der Formel IV bei Temperaturen von 10-100°C in einem wässrigen Reaktionsmedium bei einem pH-Wert von 1-8 durchführt.

16. Verfahren nach Anspruch 1, dadurch gekennzeichnet, dass man die Umsetzung eines Iminiumsalzes der Formel III mit einer Verbindung der Formel IV in Wasser durchführt.

17. Verfahren nach Anspruch 1, dadurch gekennzeichnet, dass man die Umsetzung eines Iminiumsalzes der Formel III mit einer Verbindung der Formel IV in einem zweiphasigen, aus Wasser und einem organischen, mit Wasser nicht mischbaren Lösungsmittel bestehenden Reaktionsmedium durchführt.

18. Verfahren nach Anspruch 1, dadurch gekennzeichnet, dass man ein 2-Nitrophenyl-N,N-dialkyldithiocarbamat der Formel II in einem

aus Wasser und einem mit Wasser nicht mischbaren Lösungsmittel bestehenden Reaktionsmedium in Gegenwart von 1-10 Mol pro Mol 2-Nitrophenyl-N,N-dialkyldithiocarbamat der Formel II Chlorwasserstoff, Schwefelsäure oder Perchlorsäure bei einer Temperatur von 10-100° C in ein Iminiumsalz der Formel III überführt, die organische Phase abtrennt und das Iminiumsalz der Formel III aus der wässrigen Phase nach Neutralisation der überschüssigen Säure in kristalliner Form isoliert und anschliessend in einem wässrigen Reaktionsmedium bei pH 1-8 und einer Temperatur von 10-100° C mit einer Verbindung der Formel IV zu einem 1,3-Benzodithiolderivat der Formel I umsetzt.

19. Verfahren nach Anspruch 1, dadurch gekennzeichnet, dass man ein 2-Nitrophenyl-N,N-dialkyldithiocarbamat der Formel II in einem aus Wasser und einem mit Wasser nicht mischbaren Lösungsmittel bestehenden Reaktionsmedium in Gegenwart von 1-10 Mol pro Mol 2-Nitrophenyl-N,N-dialkyldithiocarbamat der Formel II Chlorwasserstoff, Schwefelsäure oder Perchlorsäure, sowie in Gegenwart der stöchiometrischen bis 1,2-fach-stöchiometrischen Menge Amidosulfonsäure, Harnstoff, Hydroxylamin oder schwefelige Säure bei einer Temperatur von 10-100° C in ein Iminiumsalz der Formel III überführt, die organische Phase abtrennt und das Iminiumsalz der Formel III aus der wässrigen Phase nach Neutralisation der überschüssigen Säure in kristalliner Form isoliert und anschliessend in einem wässrigen Reaktionsmedium bei pH 1-8 und einer Temperatur von 10-100° C mit einer Verbindung der Formel IV zu einem 1,3-Benzodithiolderivat der Formel I umsetzt.

20. Verfahren nach Anspruch 1, dadurch gekennzeichnet, dass man ein 2-Nitrophenyl-N,N-dialkyldithiocarbamat der Formel II in einem aus Wasser und einem mit Wasser nicht mischbaren Lösungsmittel bestehenden Reaktionsmedium in Gegenwart von 1-10 Mol pro Mol 2-Nitrophenyl-N,N-dialkyldithiocarbamat der Formel II Chlorwasserstoff, Schwefelsäure oder Perchlorsäure bei einer Temperatur von 10-100° C in das

entsprechende Iminiumsalz der Formel III überführt, die organische Phase abtrennt und das in wässriger Lösung vorliegende Iminiumsalz der Formel III nach Zugabe von frischem organischem Lösungsmittel und Neutralisation der überschüssigen Säure in Gegenwart von Amidosulfonsäure, Harnstoff, Hydroxylamin oder schwefeliger Säure bei 10-100° C und einem pH von 1-8 mit einer Verbindung der Formel IV umsetzt.

21. Iminiumsalze der Formel

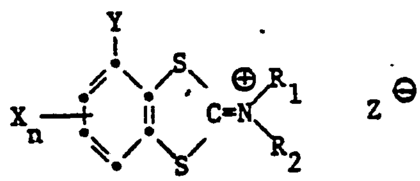 (III) ,

in welcher Y Nitro, Trifluormethyl, Cyano oder Alkylsulfonyl mit 1 bis 6 Kohlenstoffatomen in der Alkylgruppe, X Alkyl mit 1 bis 6 Kohlenstoffatomen, Alkenyl mit 2 bis 6 Kohlenstoffatomen, Nitro, Trifluormethyl, Trichlormethyl, Trifluormethoxy, Trifluormethylthio, Trifluormethylsulfinyl, Trifluormethylsulfonyl, Methoxymethyl, Cyano, Carboxy, Carbamoyl, Halogen, Amino, Acylamino, N-Alkylamino mit 1 bis 6 Kohlenstoffatomen in der Alkylgruppe, N,N-Dialkylamino mit 1 bis 6 Kohlenstoffatomen in den Alkylgruppen, N-Phenylamino, N-Alkenylamino mit 3 bis 6 Kohlenstoffatomen in der Alkenylgruppe, N,N-Dialkenylamino mit 3 bis 6 Kohlenstoffatomen in den Alkenylgruppen, n 0, 1, 2 oder 3 und $R_1$ und $R_2$ je eine Alkylgruppe mit 1 bis 6 Kohlenstoffatomen bedeuten und Z für das Anion einer starken Säure steht.

22. Verfahren zur Herstellung von Iminiumsalzen der Formel

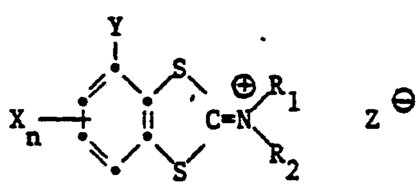 (III) ,

in welcher Y Nitro, Trifluormethyl, Cyano oder Alkylsulfonyl mit 1 bis 6 Kohlenstoffatomen in der Alkylgruppe, X Alkyl mit 1 bis 6 Kohlenstoffatomen, Alkenyl mit 2 bis 6 Kohlenstoffatomen, Nitro, Trifluormethyl, Trichlormethyl, Trifluormethoxy, Trifluormethylthio, Trifluormethylsulfinyl, Trifluormethylsulfonyl, Methoxymethyl, Cyano, Carboxy, Carbamoyl, Halogen, Amino, Acylamino, N-Alkylamino mit 1 bis 6 Kohlenstoffatomen in der Alkylgruppe, N,N-Dialkylamino mit 1 bis 6 Kohlenstoffatomen in den Alkylgruppen, N-Phenylamino, N-Alkenylamino mit 3 bis 6 Kohlenstoffatomen in der Alkenylgruppe, N,N-Dialkenylamino mit 3 bis 6 Kohlenstoffatomen in den Alkenylgruppen, n 0, 1, 2 oder 3 und $R_1$ und $R_2$ je eine Alkylgruppe mit 1 bis 6 Kohlenstoffatomen bedeuten und Z für das Anion einer starken Säure steht, dadurch gekennzeichnet, dass man ein 2-Nitrophenyl-N,N-dialkyldithiocarbamat der Formel

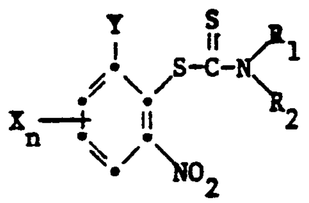

(II) ,

in welcher $R_1$, $R_2$, X, Y und n die unter Formel III angegebene Bedeutung haben, in Gegenwart einer starken Säure unter Abspaltung von salpetriger Säure cyclisiert.

23. Verfahren nach Anspruch 19, dadurch gekennzeichnet, dass man ein 2-Nitro-N,N-dialkyldithiocarbamat der Formel II in einem aus Wasser und einem mit Wasser nicht mischbaren Lösungsmittel bestehenden Reaktionsmedium in Gegenwart von 1-10 Mol pro Mol 2-Nitro-N,N-dialkyldithiocarbamat der Formel II Chlorwasserstoff, Schwefelsäure oder Perchlorsäure bei einer Temperatur von 10-100° C in das Iminiumsalz der Formel III überführt, die organische Phase abtrennt und das Iminiumsalz der Formel III aus der wässrigen Phase nach Neutralisation der überschüssigen Säure in kristalliner Form isoliert.

0036841

Europäisches Patentamt

**EUROPÄISCHER RECHERCHENBERICHT**

Nummer der Anmeldung

EP 81 81 0109

| Kategorie | EINSCHLÄGIGE DOKUMENTE<br>Kennzeichnung des Dokuments mit Angabe, soweit erforderlich, der maßgeblichen Teile | betrifft Anspruch |
|---|---|---|
| | JOURNAL OF ORGANIC CHEMISTRY Band 42, No. 7, 01-04-1977, Washington, DC, USA<br>K. RASHEED et al. "The thermal cyclization of dinitrophenyl N,N-dimethyldithiocarbamates. A novel synthesis of 1,3-benzo-dithiol-2-ones" Seiten 1265-1266<br>* Seiten 1265-1266 *<br>-- | 1 |
| | DE - A - 1 768 874 (FARBEN-FABRIKEN BAYER AG)<br>* Seiten 8-9 *<br>-- | 1 |
| D | DE - A - 2 834 061 (THE ANSUL CO.)<br>* Ansprüche *<br>-- | 1 |
| D | DE - A - 2 644 036 (THE ANSUL CO.)<br>* Ansprüche *<br>-- | 1 |
| D | JOURNAL OF ORGANIC CHEMISTRY Band 44, No. 2, 19-01-1979, Washington, DC, USA<br>K. RASHEED et al. "Study of the thermal decomposition of dinitro-phenyl N,N-dialkyldithiocarbamates and related compounds", Seiten 267-274<br>* Seite 268 *<br>-- | 1 |
| P | EP - A - 0 018 578 (CIBA-GEIGY)<br>* Patentansprüche *<br>---- | 1 |

KLASSIFIKATION DER ANMELDUNG (Int. Cl.³)

C 07 D 339/06
A 01 N 43/28//
C 07 C 155/08

RECHERCHIERTE SACHGEBIETE (Int. Cl.³)

C 07 D 339/06
A 01 N 43/28

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 12-06-1981 | BRIGHENTT |

EPA form 1503.1 06.78